# EUROPÄISCHE PATENTANMELDUNG

(11) **EP 3 042 603 A1**
(43) Veröffentlichungstag der Anmeldung: **13.07.2016**
(21) Anmeldenummer: 15400049.1
(22) Anmeldetag: 29.10.2015
(51) Int. Cl.: A61B 5/00, A61B 5/117, A61B 90/90, A61B 5/1172

(54) **VERFAHREN UND VORRICHTUNG ZUR MULTIFUNKTIONALEN MESSWERTEAUFNAHME**

(30) Priorität: 07.01.2015 DE 102015000127
(71) Anmelder: seca ag, 4153 Reinach BL 1 (CH)
(72) Erfinder: Jan, Schmidt, 22941 Bargteheide (DE)
(74) Vertreter: Patentanwälte Klickow & Partner Partnerschaftsgesellschaft mbB

(57) **Zusammenfassung**

Multifunktionale Messwerteaufnahmevorrichtung (20) und Verfahren zur Ermittlung und Übertragung von Patientenmesswerten an eine Anzeige- und Bedieneinheit oder Zentraleinheit (10), aufweisend eine Messwerteaufnahmevorrichtung (22) zur Messung von Körpereigenschaften und, oder Körperfunktionen, wobei wenigstens eine Abtastungsvorrichtung (30) zur Erfassung der Identifikationskodierung oder des Identifikationsmerkmals und wobei die Abtastungsvorrichtung in die multifunktionale Messwerteaufnahmevorrichtung (20) integriert ist, sodass eine fehlerreduzierte Zuordnung oder Vertauschung von Identifikationskodierung oder Identifikationsmerkmal und der jeweiligen Körpereigenschaft und, oder Körperfunktion unterstützt wird.

## Beschreibung

Die Erfindung betrifft eine multifunktionale Messwerteaufnahmevorrichtung und ein Verfahren zur Ermittlung und Übertragung von Patientenmesswerten an eine Anzeige- und Bedieneinheit oder Zentraleinheit, aufweisend eine Messwerteaufnahmevorrichtung zur Messung von Körpereigenschaften und, oder Körperfunktionen.

Zur Messung von Körpereigenschaften und Körperfunktionen von Subjekten, z.B. menschlichen Personen oder Tieren, werden sowohl mobile als auch stationäre Messgeräte eingesetzt. Bei den zu messenden Eigenschaften oder Funktionen handelt es sich häufig um Bio- bzw. Körperimpedanzen, Herzfunktionen, Nervenfunktionen oder Elektrokardiogrammen. Weitere Messgrößen in diesem Zusammenhang können Körpergewicht, Körperzusammensetzungen, geometrische Abmessungen, Fingerabdrücke, Körpertemperaturen an verschiedenen Stellen, Zusammensetzung des verwerteten Atemgases, Atemgasvolumina, Blutdruckwerte usw. sein. Für diese Messungen ist es erforderlich, dass das Messgerät mit dem zu messenden Subjekt verbunden wird und, oder dass die jeweiligen Messgrößen in geeigneter Weise gemessen oder erfasst und häufig auch verarbeitet und weitergeleitet werden. Ob eine Messung durch Kontakt mit dem Patienten oder kontaktlos erfolgen kann, ist abhängig von der Körpereigenschaft, der Körperfunktion bzw. dem Vitalparameter der gemessen werden soll und der dazu verwendeten Messmethode.

Die verfügbare Messtechnik generell und insbesondere in der Medizintechnik erlaubt heute, dass immer mehr Vitalparameter, verschiedene Körpereigenschaften und Körperfunktionen in immer kürzerer Zeit ermittelt und elektronisch weiterverarbeitet werden können.

Damit die Uhtersuchungskosten gering bleiben, sind wirtschaftlich optimierte Verarbeitungs- und Auswerfungsprozesse der jeweiligen Messgrößen erforderlich. Um dieses Ziel zu erreichen, werden insbesondere in der Anwendung von Krankenhäusern und großen Gemeinschaftspraxen, in denen sehr viele Messwerte von einer Vielzahl von Patienten aufgenommen werden müssen, nicht mehr Vorrichtungskombinationen bestehend aus einer Messwerteaufnahmevorrichtung und einer zugeordneten Messwertverarbeitungs- und Auswertevorrichtung eingesetzt, sondern es werden durch eine Vielzahl von Messwerteaufnahmevorrichtungen die aufgenommenen Messwerte einer meist zentralen Messwertverarbeitungs- und Auswertevorrichtung zugeleitet.

Dieses Vorgehen ist eine Konsequenz aus der Tatsache, dass immer mehr Funktionalitäten von intelligenten Sensoren ohne komplizierte Anzeige- und Bedieneinheiten ausgeführt werden müssen und statteiherdezentraten Messwertverarbeitung durch einzelne Messgeräte vermehrt zentrale Auswertungs-, Anzeige- und Bedieneinheiten mit entsprechender Vernetzung eingesetzt werden. Dabei müssen die als Satelliten bezeichneten Messwerteaufnahmevorrichtungen eindeutig einem Patienten zuordbar sein.

Für eine spätere Auswertung der Messdaten ist es erforderlich, dass eine eindeutige Zuordnung sowohl des Patienten als auch der verwendeten Messeinrichtung zu einem bestimmten Datensatz erfolgt. Für die Diagnose und Festlegung der geeigneten Behandlung zur Heilung der Krankheit des jeweiligen Patienten ist eine absolut korrekte Zuordnung unabdingbar, eine fehlerhafte Datenbasis hinsichtlich Inhalt oder Zuordnung kann äußerst negative Auswirkungen nach sich ziehen.

Es kann zum einen die Situation vorliegen, dass die gleiche Messwerteaufnahmevorrichtung nacheinander an unterschiedlichen Patienten verwendet wird, darüber hinaus kann aber auch ein bestimmter Patient in einer zeitlichen Abfolge nacheinander mit unterschiedlichen Messwerteaufnahmevorrichtungen vermessen werden. Die Durchführung der einzelnen Messungen erfolgt häufig vor dem eigentlichen Behandlungsgespräch mit dem behandelnden Arzt.

In Krankenhäusern, Großpraxen und anderen Behandlungseinrichtungen mit hohem Patientenaufkommen bzw. zu vermessenden Personen ist es ein übliches Vorgehen, die Patienten bzw. Personen mit einer Identifikationseinrichtung zu versehen, die häufig mittels eines Armbandes festgelegt wird und eine Identifizierungskodierung enthält. Bei der Identifizierungskodierung kann es sich um eine Identifikationsnummer handeln, die dem Armband als alphanumerisches Zeichen eingeprägt ist. Alternativ oder additiv zur Identifikationsnummer kann eine Identifikationskodierung als vorzugsweise maschinenlesbaren Barcode, als Farbenfolgekombination oder als eine andere geeignete Identifikationskodierung ausgebildet sein. Weiterhin kann als Identifikationsmerkmal eine der unverwechselbaren Eigenschaften des Körpers von Patienten genutzt werden wie zum Beispiel der Fingerabdruck oder die geometrisch-farblichen Merkmale der Iris eines Auges.

Wird der durch die Identifikationseinrichtung identifizierbare Patient einer Vermessung bzw. Messwerteerfassung zugeführt, erfolgt die Zuordnung der Messwerte in der Regel durch das Bedienpersonal der Messvorrichtungen oder im Fall einer maschinenlesbaren, vorzugsweise optisch oder sensorisch abtastbaren Identifikationskodierung oder dem Identifikationsmerkmal durch eine entsprechende separate Messwerteaufnahmevorrichtung.

Erfolgt die Zuordnung der Messwerte durch das Bedienpersonal der Messvorrichtungen, sind Irrtümer und fehlerhafte Messwertezuordnungen, Ablesefehler oder Vertauschungen eine immer wiederkehrende Fehlerquelle.

Erfolgt die Zuordnung der Messwerte durch eine entsprechende separate Messwerteaufnahmevorrichtung, wird dies z.B. durch einen Barcodescanner oder einen Sensor realisiert, der die dann zwingend erforderliche maschinenlesbare Identifikationskodierung oder das Identifikationsmerkmal aufnimmt. Diese Methode ist zuverlässiger als die Zuordnung der Messwerte durch das Bedienpersonal der Messvorrichtungen, aber durch die Mitwirkung menschlicher Handlungen sind auch hier fehlerhafte Zuordnungen nicht vollständig ausgeschlossen. Weitere Nachteile sind die zusätzlichen Messwerteaufnahmevorrichtungen wie z.B. Barcodescanner oder andere geeignete Sensorik, deren Verkabelung oder Drahtlosverbindung mit der Messgerätvorrichtung und der dadurch entstehende Mehraufwand erforderlicher Messungen.

Es ist daher Aufgabe der Erfindung, ein Verfahren sowie eine Vorrichtung zur Zuordnung von Messwerten zu dem jeweils vermessenen Patienten bereitzustellen, welche die Zuverlässigkeit der Messwertezuordnung generell erhöht und einen kostengünstigen Aufbau sowie einfache Handhabung unterstützt.

Erfindungsgemäß wird diese Aufgabe gelöst durch die integrale Kombination von wenigstens einer Messwerteaufnahmevorrichtung für die Messung von Körpereigenschaften und Körperfunktionen und wenigstens einer Messwerteaufnahmevorrichtung für die Abtastung der Identifikationskodierung oder des Identifikationsmerkmals sowie der Verknüpfung der jeweiligen Messwerte und Abtastergebnisse als Verfahren zur Messdatenhandhabung.

Die Erfindung erkennt, dass die Nachteile des Standes der Technik wenigstens reduziert werden können, indem der Satellit mit der wenigstens einen Messwerteaufnahmevorrichtung für die Messung von Körpereigenschaften und Körperfunktionen zusätzlich mit der wenigstens einen Messwerteaufnahmevorrichtung für die Abtastung der Identifikationskodierung oder des Identifikationsmerkmals in integraler Weise kombiniert wird. Als Sensoren eignen sich zum Beispiel integrierte Fingerabdruckscanner oder Barcodescanner oder optische Erfassungsvorrichtungen zum Beispiel Kameras.

Die Identifikationskodierung oder des Identifikationsmerkmal und die jeweilige Körpereigenschaft bzw. Körperfunktion können erfindungsgemäß gemeinsam zeitgleich oder zeitversetzt gemessen und als gemeinsames Datenpaket entweder sofort an die zentrale Bedien- und Anzeigeeinheit übertragen oder zunächst auf dem Satelliten gespeichert und für die spätere Übertragung bevorratet.

Durch die gemeinsame, zeitgleiche oder zeitversetzte Messung und gemeinsame Ablage bzw. Übertragung wird eine fehlerhafte Zuordnung oder Vertauschung von Identifikationskodierung oder Identifikationsmerkmal und die jeweilige Körpereigenschaft bzw. Körperfunktion verhindert.

Weiterer besonderer Vorteil der Kombination der wenigstens einen Messwerteaufnahmevorrichtung für die Messung von Körpereigenschaften und Körperfunktionen und der wenigstens einen Messwerteaufnahmevorrichtung für die Abtastung der Identifikationskodierung oder des Identifikationsmerkmals in integraler Weise liegt in dem Verzicht auf zusätzliche Schnittstellen, Kabelverbindungen oder weiteren Kontaktierungsgeräten.

Die erfindungsgemäße Konstruktion des Satelliten als eine praktisch multifunktionale Messwerteaufnahmevorrichtung für die Messung von Körpereigenschaften und Körperfunktionen sowie der Abtastung der Identifikationskodierung oder des Identifikationsmerkmals in integraler Weise erfolgt derart, dass im Vergleich zur Ausgangssituation, d.h. für Satelliten ausschließlich bestehend aus einer Messwerteaufnahmevorrichtung für die Messung von Körpereigenschaften und Körperfunktionen, die sicherheitstechnischen Ausstattungen nicht erhöht werden müssen. Dies wird erreicht dadurch, dass das Abtastverfahren zur Ermittlung der Identifikationskodierung oder des Identifikationsmerkmals so gewählt wird, dass es auf optischen oder kapazitiven Wirkprinzipen beruht und infolge dessen insbesondere die Schutzart des Satelliten, beispielsweise IP67, erhalten bleiben kann.

Weitere Vorteile der optischen oder kapazitiven Wirkprinzipe zur Nutzung als Abtastverfahren für die Ermittlung der Identifikationskodierung oder des Identifikationsmerkmals ist die Möglichkeit zur kostengünstigen Implementierung in einen Satelliten mit einer Messwerteaufnahmevorrichtung für die Messung von Körpereigenschaften und Körperfunktionen und deren einfacher Anwendung.

Die Erfindung umfasst sowohl eine direkte als auch eine indirekte Ermittlung und Zuordnung der Identifizierungskodierung. Dabei ist die Identifizierungskodierung in ihrer Basis eine Identifikationsnummer, die als solche sowohl maschinenverarbeitbar ist, als auch von dem Bedienungs- und Behandlungspersonal als Identifikationsgröße verwendet werden kann. Eine direkte Ermittlung der Identifizierungskodierung in Form einer Identifikationsnummer kann durch optische Messverfahren erfolgen. Bei der indirekten Ermittlung und Zuordnung der Identifizierungskodierung wird zunächst zum Beispiel ein Identifikationsmerkmal messtechnisch erfasst und anschließend dieses Merkmal der jeweils datentechnisch hinterlegten Identifikationsnummer zugeordnet.

Dazu kann beispielsweise ein Fingerabdruck, die Irisstruktur des Auges oder der Barcode auf einem Patientenarmband messtechnisch erfasst werden. Aus diesen messtechnischen Informationen wird dann die Identifikationsnummer des Patienten über eine Messwertverarbeitung ermittelt und den medizinischen Messdaten, d.h. den gemessenen Körpereigenschaften, Körperfunktionen und, oder Vitalparametern zugeordnet.

Die Erfindung unterstützt sowohl die zeitgleiche als auch die zeitversetzte Messwerteaufnahme der Messgrößen von Körpereigenschaften und Körperfunktionen im Hinblick auf die Abtastung der Identifikationskodierung und, oder des Identifikationsmerkmals.

Auch unterstützt die Erfindung sowohl die simultane, d.h. unmittelbare zeitgleiche Übertragung der Messgrößen und, oder die Abtastungsergebnisse an eine Anzeige- und Bedieneinheit bzw. Zentraleinheit als auch eine zeitversetzte Übertragung. Für die zeitversetzte Übertragung der Messgrößen von Körpereigenschaften und Körperfunktionen und, oder die Abtastungsergebnisse in Form von Identifikationskodierung und, oder Identifikationsmerkmal wird eine geeignete Datenspeicherungseinheit in dem Satelliten vorgesehen.

Der Datentransfer zwischen Satellit und Anzeige- und Bedieneinheit bzw. Zentraleinheit kann auf verschiedenen leitungsbasierenden oder drahtlosen Verfahren beruhen, beispielsweise elektrisch, optisch, Funk, WLAN, Netzwerk, Bluetooth usw.

Die Erfindung wird nachfolgend mit Bezug auf die Figuren erläutert. Im Einzelnen zeigen
- Fig. 1: ein Beispiel einer Abtastvorrichtung (30) in Form eines kompakten Moduls zum Scannen von Barcodes,
- Fig. 2: ein Beispiel einer Abtastvorrichtung (30) in Form eines kompakten Kamera-Moduls,
- Fig. 3: ein Beispiel einer Abtastvorrichtung (30) in Form eines kompakten Moduls zum Scannen des Fingerabdruckes,
- Fig. 4: beispielhaft das erfindungsgemäße Konzept bestehend aus einer Anzeige- und Bedieneinheit (10) und davon abgesetzten, d.h. physisch getrennten Messelektronik in Form eines Satelliten (20) und
- Fig. 5: beispielhaft das Konzept eines Satelliten (20) ausgebildet als multifunktionale Messwerteaufnahmevorrichtung für die Messung von Körpereigenschaften und Körperfunktionen sowie der Abtastung der Identifikationskodierung oder des Identifikationsmerkmals in integraler Weise bestehend im Wesentlichen aus einer Messmatte (21) aufweisend eine Messwerteaufnahmevorrichtung (22) zur Messung von Körpereigenschaften und Körperfunktionen und einer Abtastungsvorrichtung (30) zur Erfassung der Identifikationskodierung oder des Identifikationsmerkmals.

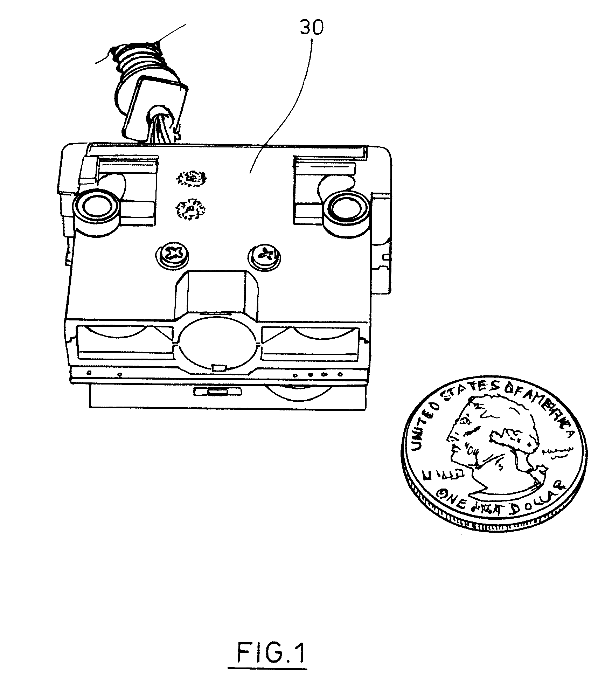

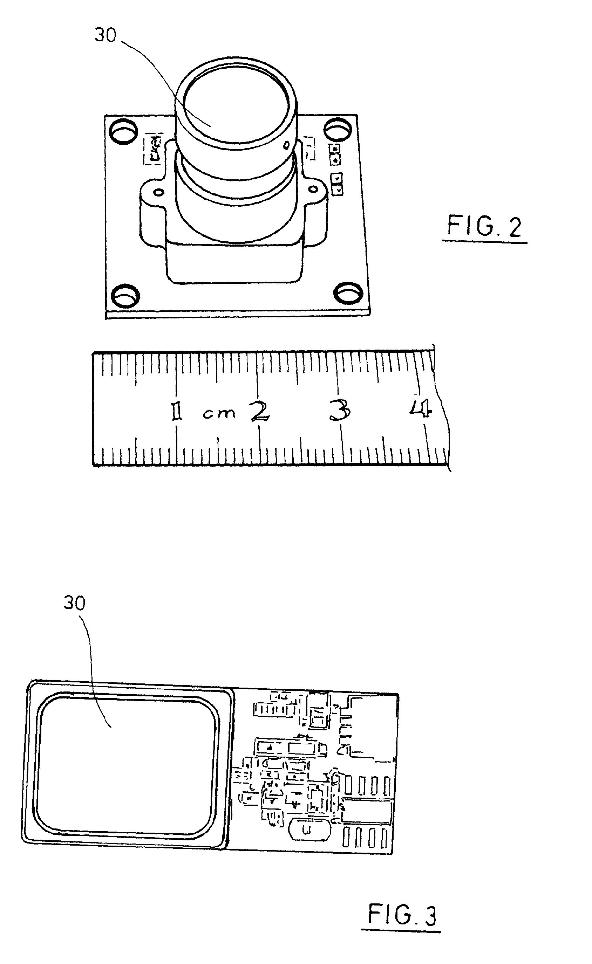

Figur 1, 2 und 3 zeigen exemplarisch mögliche Ausgestaltungen der Abtastungsvorrichtung (30) zur Erfassung der Identifikationskodierung oder des Identifikationsmerkmals in Form von Kompaktmodulen basierend auf optischen oder kapazitiven Wirkprinzipen.

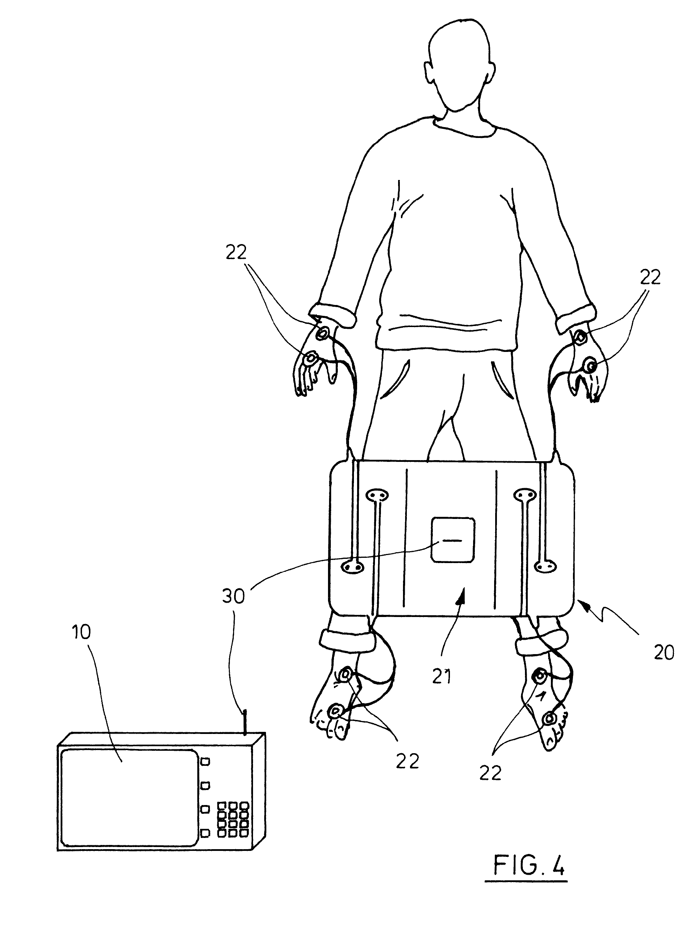

Figur 4 zeigt beispielhaft das Konzept bestehend aus einer Anzeige- und Bedieneinheit (10) und davon abgesetzten, d.h. physisch getrennten Messelektronik in Form eines Satelliten (20).
Mit diesem Konzept wird der erforderliche, wirtschaftlich optimierte Verarbeitungs- und Auswertungsprozess der jeweiligen Messgrößen erreicht. Insbesondere in der Anwendung im Rahmen sehr vieler aufzunehmender Messwerte können auf diese Weise und mittels einer Vielzahl von Satelliten (20) die aufgenommenen Messwerte einer meist zentralen Messwertverarbeitungs- und Auswertevorrichtung (10) zugeleitet werden. Durch dieses Konzept wird der Tatsache Rechnung getragen, dass immer mehr Funktionalitäten von intelligenten Sensoren ohne komplizierte Anzeige- und Bedieneinheiten ausgeführt werden müssen und statt einer dezentralen Messwertverarbeitung durch einzelne Messgeräte vermehrt zentrale Auswertungs-, Anzeige- und Bedieneinheiten mit entsprechender Vernetzung eingesetzt werden.

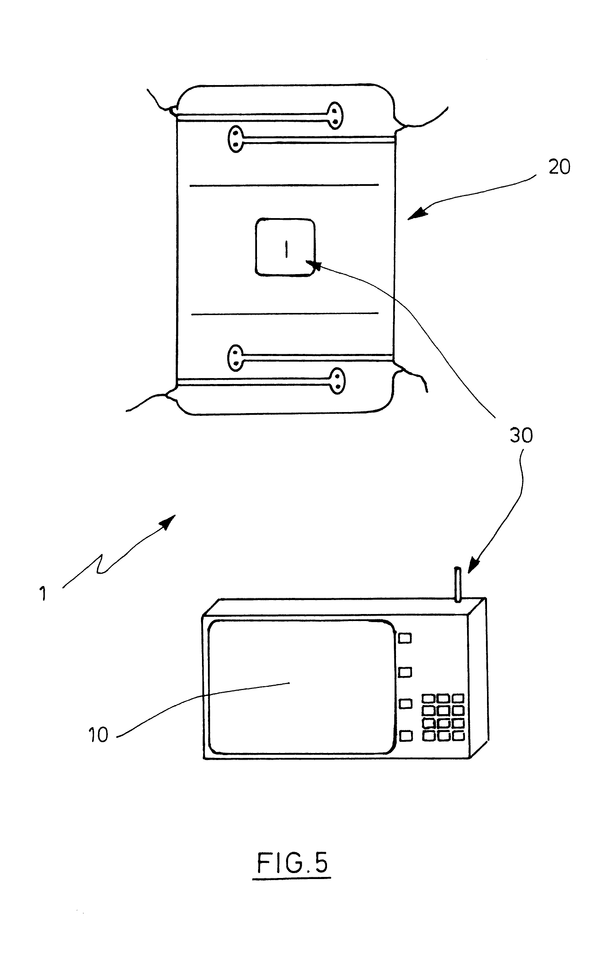

Figur 5 illustriert beispielhaft das erfindungsgemäße Konzept eines Satelliten (20) ausgebildet als multifunktionale Messwerteaufnahmevorrichtung für die Messung von Körpereigenschaften und Körperfunktionen sowie der Abtastung der Identifikationskodierung oder des Identifikationsmerkmals in integraler Weise bestehend im Wesentlichen aus einer Messmatte (21) aufweisend eine Messwerteaufnahmevorrichtung (22) zur Messung von Körpereigenschaften und Körperfunktionen und einer Abtastungsvorrichtung (30) zur Erfassung der Identifikationskodierung oder des Identifikationsmerkmals. Üblicherweise ist eine Abtastungsvorrichtung (30) zur Erfassung der Identifikationskodierung oder des Identifikationsmerkmals pro Satellit (20) ausreichend, Figur 5 zeigt verschiedene Positionierungsmöglichkeiten auf.

Der Satellit (20) als multifunktionale Messwerteaufnahmevorrichtung kann auf verschiedenen Vorrichtungen zur Messung von Körpereigenschaften und Körperfunktionen basieren, in die die Vorrichtung zur Abtastung (30) der Identifikationskodierung oder des Identifikationsmerkmals erfindungsgemäß integriert wird. Die in Figur 5 beispielhaft gezeigte Messmatte (21) als Basis für den Satelliten (20) bietet Handhabungsvorteile. Da die Messmatte (21) während der Messwertaufnahme quer über den Beinen des Patienten liegt, sind die Enden der Messmatte (21) mit ihrer bevorzugten Positionierung wenigstens einer Abtastungsvorrichtung (30) zur Erfassung der Identifikationskodierung oder des Identifikationsmerkmals sehr leicht durch den Patienten erreichbar. Ein Einscannen der Identifikationskodierung von dem Patientenarmband oder die Erfassung eines Identifikationsmerkmals, zum Beispiel dem Fingerabdruck, ist in einfacher Weise und insbesondere unter aktiver Mithilfe des Patienten möglich. Gemäß einer Variante der Erfindung werden die Messdaten mit einem von der Personenidentifikation abgeleiteten Code verschlüsselt, z.B. rückgekoppeltes, insbesondere linear rückgekoppeltes, Schieberegister. Die Entschlüsselung der personenbezogenen Messdaten ist auf einem beliebigen Zielsystem nur dann möglich, wenn die korrekte Personenidentifikation bekannt ist. Damit ist sichergestellt, dass die Messdaten korrekt zugeordnet werden. Ein Ablegen der Personenidentifikation in dem Messdatensatz wäre in diesem Fall nicht nötig.

Gemäß einer weiteren Variante des Erfinders können die zu messenden Eigenschaften auch prinzipiell Bilder des Patienten sein, um z.B. durch den Arzt oder eine geeignete SW die Proportionen beurteilen zu lassen. Auch 3D-Kameras bzw. deren Bilder können dazu passen.

Insbesondere ist vorgesehen, dass Schritt b.) durch eine indirekte Ermittlung der Identifikationsnummer derart erfolgt, dass eine Identifizierungskodierung ermittelt wird, die einer Identifikationsnummer zuordbar ist.

Insbesondere ist vorgesehen, dass Schritt c. auf leitungsbasierenden oder drahtlosen Verfahren realisiert ist.

Insbesondere ist vorgesehen, dass die Messdaten mit einem von der Personenidentifikation abgeleiteten Code verschlüsselt werden, insbesondere durch ein rückgekoppeltes, beispielsweise linear rückgekoppeltes, Schieberegister, wobei die Entschlüsselung der personenbezogenen Messdaten auf einem beliebigen Zielsystem nur dann möglich ist, wenn die korrekte Personen-identifikation bekannt ist.

## Patentansprüche

1. Multifunktionale Messwerteaufnahmevorrichtung (20) zur Ermittlung und Übertragung von Patientenmesswerten an eine Anzeige- und Bedieneinheit oder Zentraleinheit aufweisend eine Messwerteaufnahmevorrichtung (22) zur Messung von Körpereigenschaften und, oder Körperfunktionen, **gekennzeichnet durch** wenigstens eine Abtastungsvorrichtung (30) zur Erfassung der Identifikationskodierung oder des Identifikationsmerkmals, wobei die Abtastungsvorrichtung (30) in die multifunktionale Messwerteaufnahmevorrichtung (20) integriert ist, sodass eine fehlerreduzierte Zuordnung oder Vertauschung von Identifikationskodierung oder Identifikationsmerkmal und der jeweiligen Körpereigenschaft und, oder Körperfunktion unterstützt wird.

2. Multifunktionale Messwerteaufnahmevorrichtung (20) nach Anspruch 1, **dadurch gekennzeichnet, dass** die multifunktionale Messwerteaufnähmevorrichtung (20) als Satellit ausgebildet ist, sodass die autarke Messung von Körpereigenschaften und, oder Körperfunktionen und die Erfassung der Identifikationskodierung oder des Identifikationsmerkmals unterstützt ist.

3. Multifunktionale Messwerteaufnahmevorrichtung (20) nach Anspruch 1, **dadurch gekennzeichnet, dass** die Messwerteaufnahmevorrichtung (22) zur Messung von Körpereigenschaften und, oder Körperfunktionen und die wenigstens eine Abtastungsvorrichtung (30) zur Erfassung der Identifikationskodierung oder des Identifikationsmerkmals in eine Messmatte (21) integriert sind.

4. Multifunktionale Messwerteaufnahmevorrichtung (20) nach Anspruch 3, **dadurch gekennzeichnet, dass** die wenigstens eine Abtastungsvorrichtung (30) zur Erfassung der Identifikationskodierung oder des Identifikationsmerkmals in einem Randbereich der Messmatte (21) positioniert ist.

5. Multifunktionale Messwerteaufnahmevorrichtung (20) nach Anspruch 1, **dadurch gekennzeichnet, dass** die wenigstens eine Abtastungsvorrichtung (30) zur Erfassung der Identifikationskodierung oder des Identifikationsmerkmals derart ausgebildet ist, dass die sicherheitstechnische Ausstattung der multifunktionale Messwerteaufnahmevorrichtung (20) basierend auf der Messwerteaufnahmevorrichtung (22) zur Messung von Körpereigenschaften und, oder Körperfunktionen auch die Abtastvorrichtung (30) umfasst.

6. Multifunktionale Messwerteaufnahmevorrichtung (20) nach Anspruch 1, **dadurch gekennzeichnet, dass** die wenigstens eine Abtastungsvorrichtung (30) zur Erfassung der Identifikationskodierung oder des Identifikationsmerkmals auf einem optischen oder kapazitiven Wirkprinzip basiert.

7. Multifunktionale Messwerteaufnahmevorrichtung (20) nach Anspruch 1, **dadurch gekennzeichnet, dass** die wenigstens eine Abtastungsvorrichtung (30) zur Erfassung der Identifikationskodierung oder des Identifikationsmerkmals als Sensor ausgebildet ist.

8. Multifunktionale Messwerteaufnahmevorrichtung (20) nach Anspruch 1, **dadurch gekennzeichnet, dass** der wenigstens eine Sensor der Abtastungsvorrichtung (30) zur Erfassung der Identifikationskodierung oder des Identifikationsmerkmals ein Fingerabdruckscanner oder ein Barcodescanner oder eine Kamera ist.

9. Multifunktionale Messwerteaufnahmevorrichtung (20) nach Anspruch 1, **dadurch gekennzeichnet, dass** wenigstens eine Datenspeichervorrichtung zur temporären oder dauerhaften Speicherung von Messwerten der Messwerteaufnahmevorrichtung (22) und, oder der wenigstens einen Abtastungsvorrichtung (30) vorgesehen ist.

10. Verfahren zur Ermittlung und Übertragung von Patientenmesswerten an eine Anzeige- und Bedieneinheit oder Zentraleinheit aufweisend die Schritte
a.) Messen der Körpereigenschaften und, oder Körperfunktionen eines Patienten,
b.) Erfassung der Identifikationskodierung oder des Identifikationsmerkmals des zu vermessenden Patienten,
c.) Übertragung der gemessenen und erfassten Daten an eine Anzeige- und Bedieneinheit oder Zentraleinheit,
**dadurch gekennzeichnet, dass** die gemessenen und erfassten Daten zu einem Datenpaket verknüpft werden, sodass eine fehlerreduzierte Zuordnung oder Vertauschung von Identifikationskodierung oder Identifikationsmerkmal und der jeweiligen Körpereigenschaft und, oder Körperfunktion unterstützt wird.

11. Verfahren zur Ermittlung und Übertragung von Patientenmesswerten an eine Anzeige- und Bedieneinheit oder Zentraleinheit nach Anspruch 10, **dadurch gekennzeichnet, dass** die Schritte a.) und b.) zeitgleich erfolgen.

12. Verfahren zur Ermittlung und Übertragung von Patientenmesswerten an eine Anzeige- und Bedieneinheit oder Zentraleinheit nach Anspruch 10, **dadurch gekennzeichnet, dass** die Schritte a.) und b.) zeitversetzt zueinander erfolgen.

13. Verfahren zur Ermittlung und Übertragung von Patientenmesswerten an eine Anzeige- und Bedieneinheit oder Zentraleinheit nach Anspruch 10, **dadurch gekennzeichnet, dass** der Schritt c.) simultan zu den Schritten a.) und, oder b.) erfolgt.

14. Verfahren zur Ermittlung und Übertragung von Patientenmesswerten an eine Anzeige- und Bedieneinheit oder Zentraleinheit nach Anspruch 10, **dadurch gekennzeichnet, dass** der Schritt c.) zeitversetzt zu den Schritten a.) und, oder b.) erfolgt, wobei die wenigstens eine Datenspeichervorrichtung der multifunktionalen Messwerteaufnahmevorrichtung (20) die Messwerte der Messwerteaufnahmevorrichtung (22) und, oder der wenigstens einen Abtastungsvorrichtung (30) temporär oder dauerhaft speichert.

15. Verfahren zur Ermittlung und Übertragung von Patientenmesswerten an eine Anzeige- und Bedieneinheit oder Zentraleinheit nach Anspruch 10, **dadurch gekennzeichnet, dass** Schritt b.) durch eine direkte Ermittlung der Identifikationsnummer erfolgt.
